# EUROPEAN PATENT APPLICATION

(11) **EP 3 311 768 A1**
(43) Date of publication of application: **25.04.2018**
(21) Application number: 16811333.0
(22) Date of filing: 28.04.2016
(51) Int. Cl.: A61B 34/37, B25J 3/00

(54) **MEDICAL SYSTEM**

(30) Priority: 18.06.2015 JP 2015122943
(71) Applicant: Olympus Corporation, Hachioji-shi Tokyo 192-8507 (JP)
(72) Inventor: NICHOGI, Masao, Tokyo 192-8507 (JP)
(74) Representative: Gunzelmann, Rainer
(86) International application number: PCT/JP2016/063460
(87) International publication number: WO 2016/203858

(57) **Abstract**

The present invention can cope with both localized treatment and large-scale invasive surgery and reduces operating time. Provided is a medical system (1) comprising: a first robot arm (3) provided with a first manipulator (12) including a first treatment section (15) and with a first imaging unit (14) having a field of view that includes a movable range of the first treatment section (15); a second imaging unit (10) having a field of view that is wider than said field of view; a display unit (20) that displays video from the first or second imaging unit (10, 14); a switching unit (22) that switches the video; an operation unit (6) for inputting operation command for the first manipulator (12) and the first robot arm (3); and a control unit (7) that controls the first manipulator (12) and the first robot arm (3) on the basis of the operation command. The control unit (7) controls the first robot arm (3) and the first manipulator (12) according to the operation command when the video is switched to that from the second imaging unit (10), and controls the first manipulator (12) according to the operation command when the video is switched to that from the first imaging unit (14) .

## Description

### {Technical Field}

The present invention relates to a medical system.

### {Background Art}

In order to perform a surgical operation on a patient with a plurality of manipulators, in the related art there is a known surgical operation system in which, with two manipulators and an endoscope that are introduced inside the body from a single entry port via a guide tube, treatment is performed with the two manipulators while capturing images of an affected area with a local field of view (for example, see Patent Literature 1).

### {Citation List}

### {Patent Literature}

{PTL 1}
Japanese Translation of PCT International Application, Publication No. 2009-539573

### {Summary of Invention}

### {Technical Problem}

However, with the surgical operation system in Patent Literature 1, since the movable ranges of the two manipulators are restricted to the field of view of the endoscope, and the field of view of the endoscope is narrow, there is a drawback in that it is only possible to perform treatment in a small area. By moving the distal end of the guide tube, with the entry port serving as a fulcrum, it is possible to move the field of view of the endoscope and the movable ranges of the manipulators themselves; however, when performing large-scale invasive surgery, such as in the initial stage of an operation (to secure an operative field), there is a problem in that, when moving the manipulators in directions that cannot be seen, there is insufficient information about the surroundings, and the manipulators must be moved carefully and slowly while avoiding interference with the surrounding tissue, which takes time.

The present invention has been conceived in light of the circumstances described above, and an object thereof is to provide a medical system that can cope with both localized treatment and large-scale invasive surgery and that can reduce operating time.

### {Solution to Problem}

In order to achieve the above object, the present invention provides the following solutions.

An aspect of the present invention provides a medical system including: a first robot arm provided, at a distal end thereof, with a first manipulator including a first treatment section at a distal end thereof and with a first imaging unit having a local field of view that includes a movable range of the first treatment section moved by the first manipulator; a second imaging unit having a global field of view that is wider than the local field of view of the first imaging unit; a display unit that displays video from the second imaging unit or the first imaging unit; a switching unit that switches the video displayed on the display unit; an operation unit for inputting an operation command for the first manipulator and the first robot arm; and a control unit that controls the first manipulator and the first robot arm on the basis of the operation command input via the operation unit, wherein the control unit controls the first robot arm and the first manipulator according to the operation command input via the operation unit when the switching unit switches to the video from the second imaging unit, and controls the first manipulator according to the operation command input via the operation unit when the switching unit switches to the video from the first imaging unit.

According to this aspect, when the video displayed on the display unit is switched, by operating the switching unit, to the video of the local field of view obtained by the first imaging unit, the control unit controls the first manipulator, which is moved in the local field of view, on the basis of the operation command from the operation unit. On the other hand, when the video displayed on the display unit is switched, by operating the switching unit, to the video of the global field of view obtained by the second imaging unit, the control unit controls the first robot arm, which is moved in the global field of view, on the basis of the operation command from the operation unit.

Accordingly, in large-scale invasive surgery, the operator uses the switching unit to switch the field of view to the global field of view of the second imaging unit so that large movement of the treatment section can be performed by moving the first robot arm and the first manipulator while checking, on the display unit, the state of the surroundings of the first robot arm in a wide field of view. On the other hand, when performing treatment of the affected area, the operator uses the switching unit to switch the field of view to the local field of view of the first imaging unit so that intricate treatment can be performed.

The above-described aspect may further include a second robot arm provided with a second treatment section at a distal end thereof and having a movable range of the second treatment section within the global field of view, wherein the control unit may control the first robot arm, the first manipulator, and the second robot arm according to the operation command input via the operation unit when the switching unit switches to the video from the second imaging unit.

By doing so, in large-scale invasive surgery, the operator uses the switching unit to switch the field of view to the global field of view of the second imaging unit so that it is possible to perform large movement of the first treatment section by moving the first robot arm and the first manipulator and to perform large movement of the second treatment section by moving the second robot arm, while checking, on the display unit, the state of the surroundings of the first robot arm and the second robot arm.

The above-described aspect may further include, at a distal end of the first robot arm, a second manipulator provided with a third treatment section at a distal end thereof and having a movable range of the third treatment section within the local field of view, wherein the control unit may control the first manipulator and the second manipulator according to the operation command input via the operation unit when the switching unit switches to the video from the first imaging unit.

By doing so, when performing treatment on the affected area, the operator uses the switching unit to switch the field of view to the local field of view of the first imaging unit so as to move the first treatment section of the first manipulator and the third treatment section of the second manipulator while checking the video from the first imaging unit, which is displayed on the display unit, so that intricate treatment can be performed with both arms.

In the above-described aspect, the operation unit may be provided with a first operation input section that is operated by one hand of an operator and a second operation input section that is operated by the other hand of the operator; and when the switching unit switches to the video from the second imaging unit, the control unit may control the first robot arm and the first manipulator according to the operation command input via the first operation input section, and may control the second robot arm according to the operation command input via the second operation input section.

By doing so, the operator can operate the first robot arm and first manipulator and the second robot arm, which correspond to his/her two hands, respectively, so that it is possible to perform global treatment with both arms also in the state where the video of the global field of view is displayed on the display unit.

In the above-described aspect, when the switching unit switches to the video from the first imaging unit, the control unit may control the first manipulator according to the operation command input via the first operation input section, and may control the second manipulator according to the operation command input via the second operation input section.

By doing so, the operator can operate the first manipulator and the second manipulator, which correspond to his/her two hands, respectively, so that it is possible to perform intricate treatment with both arms in the state where the video of the local field of view is displayed on the display unit.

When the switching unit switches to the video from the second imaging unit, the control unit may make a coordinate system of the operation unit coincide with a fixed coordinate system in the second imaging unit.

By doing so, when operating the operation unit while looking at the video from the second imaging unit, which is operated on the display unit, the operator can perform intuitive operations. In other words, when an operation command instructing movement in one direction is input via the operation unit, the first robot arm, the second robot arm, the first manipulator, or the second manipulator displayed on the display unit can be moved on the display unit in the same direction as the operation unit.

In the above-described aspect, when the switching unit switches to the video from the first imaging unit, the control unit may make the coordinate system of the operation unit coincide with a fixed coordinate system in the first imaging unit.

By doing so, when operating the operation unit while looking at the video from the first imaging unit, which is operated on the display unit, the operator can perform intuitive operations. In other words, when an operation command instructing movement in one direction is input via the operation unit, the first manipulator or the second manipulator displayed on the display unit can be moved on the display unit in the same direction as the operation unit.

### {Advantageous Effects of Invention}

The present invention affords the advantage that it can cope with both localized treatment and large-scale invasive surgery and can reduce operating time.

### {Brief Description of Drawings}

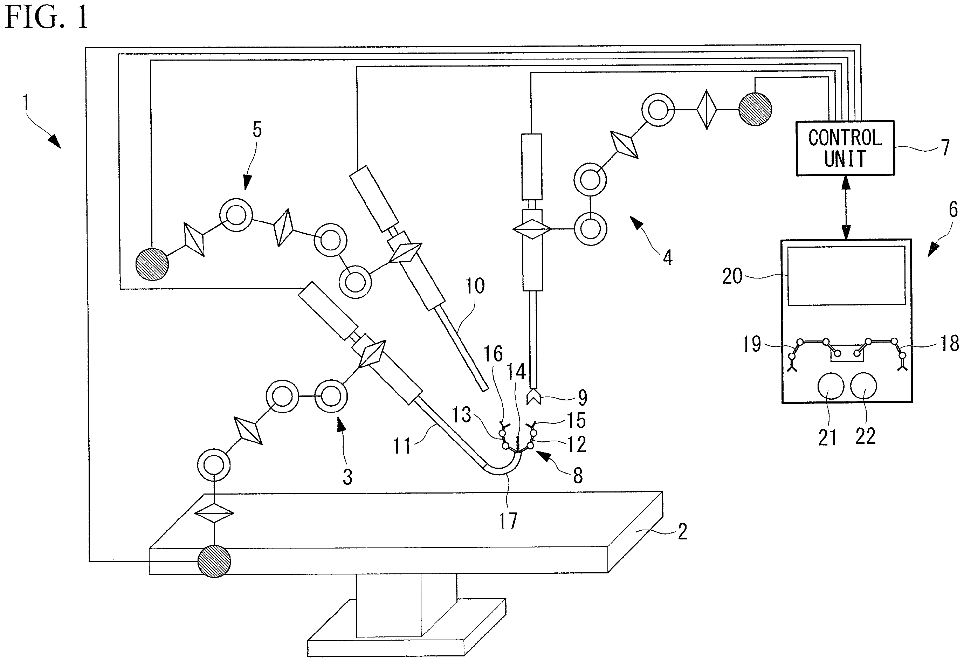

{Fig. 1}
   Fig. 1 is a diagram showing the overall configuration of a medical system according to an embodiment of the present invention.
{Fig. 2}
   Fig. 2 is a diagram showing an operation unit and a monitor display example in an overview mode in the medical system in Fig. 1.
{Fig. 3}
   Fig. 3 is a diagram showing the operation unit and a monitor display example in a local mode in the medical system in Fig. 1.
{Fig. 4}
   Fig. 4 is a diagram showing the overall configuration of a first modification of the medical system in Fig. 1.
{Fig. 5}
   Fig. 5 is a diagram showing an operation unit and a monitor display example in an overview mode in the medical system in Fig. 4.
{Fig. 6}
   Fig. 6 is a diagram showing the operation unit and a monitor display example in a local mode in the medical system in Fig. 4.
{Fig. 7}
   Fig. 7 is a diagram showing the overall configuration of a second modification of the medical system in Fig. 1.
{Fig. 8}
   Fig. 8 is a diagram showing the overall configuration of a third modification of the medical system in Fig. 1.

### {Description of Embodiment}

A medical system 1 according to an embodiment of the present invention will be described below with reference to the drawings.

As shown in Fig. 1, the medical system 1 according to this embodiment is provided with three robots 3, 4 and 5 that are disposed in the vicinity of a bed 2 on which a patient reclines; an operation unit 6 that is operated by an operator; and a control unit 7 that controls the three robots 3, 4, and 5 on the basis of operation commands input by operating the operation unit 6.

The first robot 3 is a 6-axis multijointed first robot arm on which a treatment unit 8 is mounted at the distal end thereof.

The second robot 4 is a 6-axis multijointed second robot arm on which a second treatment section 9 is mounted at the distal end thereof.

The third robot 5 is a 6-axis multijointed third robot arm on which an endoscope 10, which is a second imaging unit, is mounted at the distal end thereof.

As shown in Fig. 1, the treatment unit 8 is provided with, for example, an insertion section 11 that penetrates body surface tissue so as to be inserted into the body; a first manipulator 12 and a second manipulator 13 having one or more joints that protrude forward from the distal end of the insertion section 11; and a first imaging unit 14 that protrudes forward from the distal end of the insertion section 11. The first manipulator 12 is provided with a first treatment section 15 at the distal end thereof. The second manipulator 13 is provided with a third treatment section 16 at the distal end thereof. The insertion section 11 is provided with a bending section 17 at the end thereof, and by moving the bending section 17, it is possible to change the orientations of the first manipulator 12, the second manipulator 13, and the first imaging unit 14.

The first imaging unit 14 has a local field of view including the movable ranges of the first manipulator 12 and the second manipulator 13. In other words, the first manipulator 12 and the second manipulator 13 disposed at the distal end of the insertion section 11 of the treatment unit 8 always appear in the video of the small local field of view captured by the first imaging unit 14, so that it is possible to perform intricate treatment on an affected area captured at comparatively high magnification.

The endoscope 10 has a global field of view larger than that of the first imaging unit 14. Accordingly, it is possible to observe a comparatively wide area including the movable ranges of the first treatment section 15 and the third treatment section 16 supported by the first manipulator 12 and the second manipulator 13, due to the movement of the first robot arm 3, and the movable range of the second treatment section 9 supported on the distal end of the second robot arm 4.

The third robot 5 is not moved after the global field of view of the endoscope 10 is determined by initial settings.

As shown in Fig. 1, the operation unit 6 is provided with a first operation input section 18 that is operated by the right hand of the operator and a second operation input section 19 that is operated by the left hand of the operator. The operation input sections 18 and 19 each have a multijointed structure so that the positions and orientations of the treatment sections 9, 15, and 16 disposed at the distal ends can be arbitrarily specified in three dimensions.

The operation unit 6 is provided with a monitor (display unit) 20 that displays video obtained by the first imaging unit 14 or the endoscope 10. The operation unit 6 is provided with: a clutch 21 that switches between engagement and disconnection of each of the robot arms 3, 4, and 5 and the first and second operation input sections 18 and 19; and a switching switch (switching unit) 22 for switching the video to be displayed on the monitor 20 between the video from the first imaging unit 14 and the video from the endoscope 10.

When the video obtained by the first imaging unit 14 is selected so as to be displayed on the monitor 20 by the operation of the switching switch 22, the control unit 7 image processes the video obtained by the first imaging unit 14 and sends it to the monitor 20, and also performs control so as to make the fixed coordinate system in the first imaging unit 14 coincide with the coordinate system of the operation unit 6 (local mode).

Thus, in this case, the control unit 7 moves the first manipulator 12 and the second manipulator 13, which appear in the video of the local field of view obtained by the first imaging unit 14, according to the operation command input at the operation unit 6.

In other words, the control unit 7 uses, for example, a prescribed simultaneous transformation matrix, to perform a coordinate transformation from a motion vector in the coordinate system of the operation unit 6 to a motion vector in a coordinate system in which appropriate hand-eye coordination with respect to the first imaging unit 14 can be realized. Accordingly, the control unit 7 performs control so as to move the first manipulator 12 in the video displayed on the monitor 20 in the same direction as the first operation input section 18, on the basis of the operation command input via the first operation input section 18 in the operation unit 6. Similarly, the control unit 7 performs control so as to move the second manipulator 13 in the video displayed on the monitor 20 in the same direction as the second operation input section 19, on the basis of the operation command input via the second operation input section 19 in the operation unit 6.

When the video obtained by the endoscope 10 is selected so as to be displayed on the monitor 20 by the operation of the switching switch 22, the control unit 7 image processes the video obtained by the endoscope 10 and sends it to the monitor 20, and also performs control so as to make the fixed coordinate system in the endoscope 10 coincide with the coordinate system of the operation unit 6 (overview mode).

Thus, in this case, the control unit 7 moves the first manipulator 12 and the second treatment section 9, which appear in the video of the global field of view obtained by the endoscope 10, according to the operation command input at the operation unit 6.

In other words, by performing a coordinate transformation from a motion vector in the coordinate system of the operation unit 6 to, for example, a motion vector in a coordinate system in which appropriate hand-eye coordination with respect to the endoscope 10 can be realized, the control unit 7 performs control so as to move the first robot arm 3, the bending section of the operation unit 6, and the first manipulator (the six degrees of freedom of the joints having redundancy as a whole are set) 12 on the basis of the operation command input via the first operation input section 18 in the operation unit 6, and performs control so as to move the second robot arm 4 on the basis of the operation command input via the second operation input section 19.

The operation of the thus-configured medical system 1 according to this embodiment will be described below.

To perform treatment inside the body of the patient, for example, at an affected area in the abdominal cavity, by using the medical system 1 according to this embodiment, trocars are disposed in three holes formed so as to penetrate the abdominal wall, and the distal ends of the treatment unit 8, the endoscope 10, and the second treatment section 9 are disposed inside the abdominal cavity through each of the trocars. The movements of the three robot arms 3, 4, and 5 are restricted so that they move with the trocar positions serving as fulcrums.

To dispose the treatment unit 8, the endoscope 10, and the second treatment section 9 inside the abdominal cavity, in the state in which the clutch 21 is disengaged, the endoscope 10 inserted in the abdominal cavity is disposed at an appropriate position, and the third robot arm 5 is stopped. Then, the first robot arm 3, the second robot arm 4, and the bending section 17 of the insertion section 11 are operated so that the first manipulator 12, the second manipulator 13, the first imaging unit 14, and the second treatment section 9 are disposed in the field of view of the endoscope 10. Then, the overview mode is selected with the switching switch 22, and the video displayed on the monitor 20 is switched to the video of the global field of view obtained by the endoscope 10.

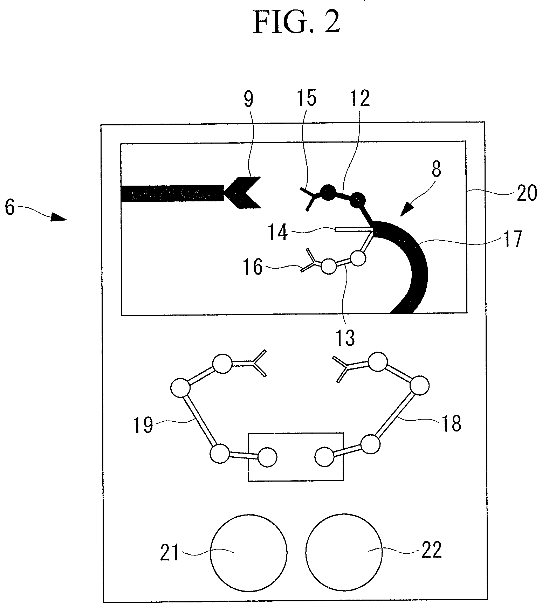

In this state, the operator moves the first operation input section 18 and the second operation input section 19 of the operation unit 6 so as to set the states of the first operation input section 18 and the second operation input section 19 of the operation unit 6 to substantially the same positional relationship of the first manipulator 12 and the second treatment section (black filled section) 9 displayed on the monitor 20, as shown in Fig. 2.

Subsequently, by engaging the clutch 21, control is commenced by the control unit 7.

In other words, when the video obtained by the endoscope 10 is displayed on the monitor 20 by means of the switching switch 22, the operation input using the first operation input section 18 is used for moving the first manipulator 12, and the operation input using the second operation input section 19 is used for moving the second treatment section 9.

As a result, when the first operation input section 18 is operated, on the basis of the operation input generated thereby, the control unit 7 moves the first treatment section 15, with the first robot arm 3, the insertion section 11, and the first manipulator 12 interlocked.

In other words, in the treatment unit 8 having the two manipulators 12 and 13, which are supported by the first robot arm 3, by driving only the first manipulator 12 at the right side as the target to be operated, it is possible to operate the second treatment section 9, which is supported by the second robot arm 4 separately from the first robot arm 3, with the second operation input section 19, which is operated with the left hand. Thus, when the operator operates the second operation input section 19, which is gripped in the left hand, on the basis of the operation input generated thereby, the control unit 7 moves the second robot arm 4 to move the second treatment section 9. As a result, in the local mode and the overview mode, the targets to be moved with the first operation input section 18 are fixed to the first manipulator 12 and the treatment section 15; however, by switching the mode with the switching switch 22, the target to be moved with the second operation input section 19 is switched to the second treatment section 9, or to the second manipulator 13 and the treatment section 16.

Because the global field of view obtained by the endoscope 10 is a wide field of view including the movable ranges of the distal ends of the second treatment section 9 and the treatment unit 8, tissue X or the like located in the vicinity of the distal ends of the second treatment section 9 and the treatment unit 8 can be displayed on the monitor 20 simultaneously. Therefore, advantages are afforded in that the operator can obtain sufficient information for avoiding interference with surrounding tissue or the like around the second treatment section 9 and the treatment unit 8, which facilitates large-scale invasive surgery and makes it possible to rapidly perform tasks such as ensuring an operative field.

Thus, in the case where the first manipulator 12 is disposed in the vicinity of an affected area, the operator selects the local mode with the switching switch 22 to switch the video displayed on the monitor 20 to the video from the first imaging unit 14. Accordingly, the control unit 7 switches to a control mode in which the first manipulator 12 and the second manipulator 13 are moved in accordance with the operation commands at the operation unit 6, and the second robot arm 4 is locked in position.

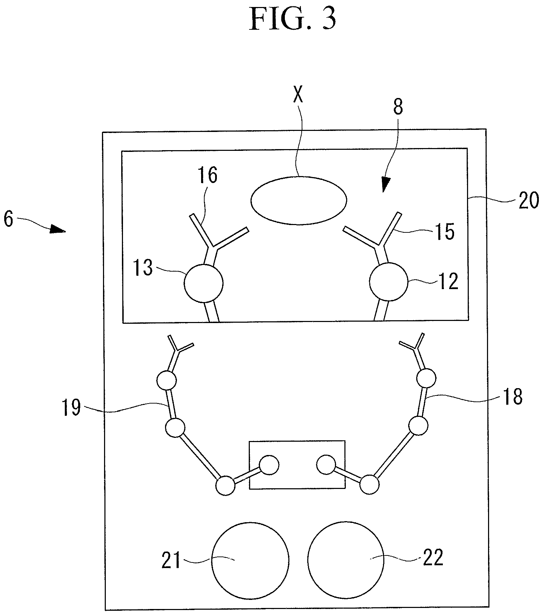

The operator disengages the clutch 21 and, as shown in Fig. 3, moves the first operation input section 18 in his/her right hand to match the state of the first manipulator 12 displayed on the monitor 20, and moves the second operation input section 19 in his/her left hand to match the state of the second manipulator 13 displayed on the monitor 20.

In this state, by engaging the clutch 21, the control unit 7 performs control so as to move the first manipulator 12 on the basis of the operation command input via the first operation input section 18 and so as to move the second manipulator 13 on the basis of the operation command input via the second operation input section 19. At this time, the first robot arm 3 and the bending section 17 of the insertion section 11 are locked so as not to move.

At this time, the control unit 7 obtains the fixed coordinate system in the first imaging unit 14 and performs control so as to make this coordinate system coincident with the coordinate system of the operation unit 6. Accordingly, since the first manipulator 12 and the second manipulator 13 can be made to move in directions matching the operating directions of the first operation input section 18 and the second operation input section 19 by movement amounts that are proportional to the operating levels thereof, operations can be performed intuitively.

Since the video from the first imaging unit 14 has a fixed angle of view due to the movements of the first robot arm 3 and the bending section 17 being locked, only the pair of manipulators 12 and 13 is movable. In other words, hand-eye-coordination ends, and the first treatment section 15 and the third treatment section 16 can be operated in a coordinated manner.

Then, because the video of the local field of view obtained by the first imaging unit 14 is displayed on the monitor 20, an advantage is afforded in that it is possible to carry out precise treatment while checking the affected area, the first manipulator 12, and the second manipulator 13 displayed in a magnified manner on the monitor 20.

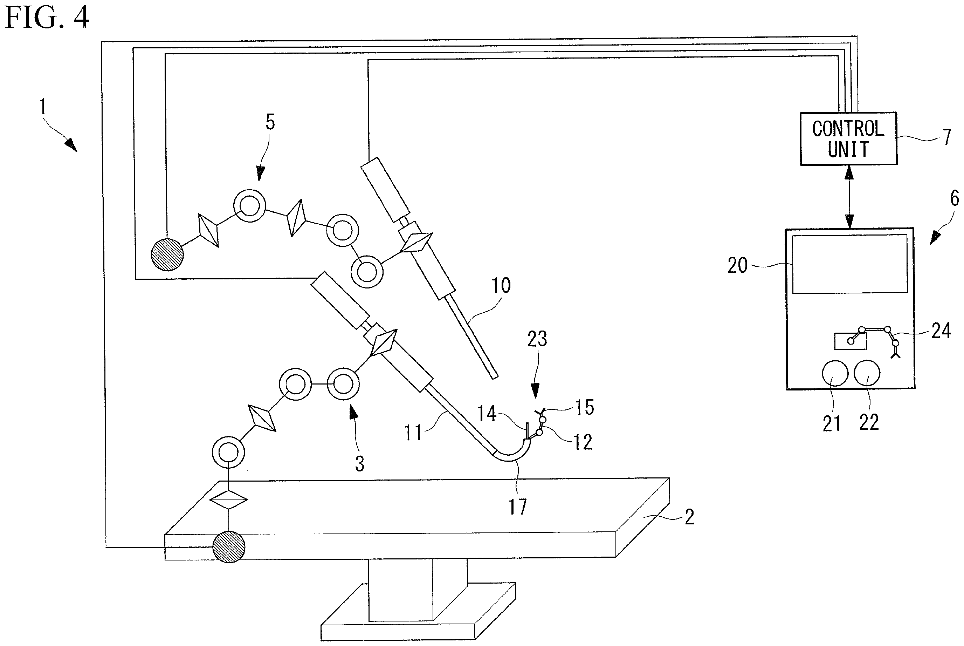

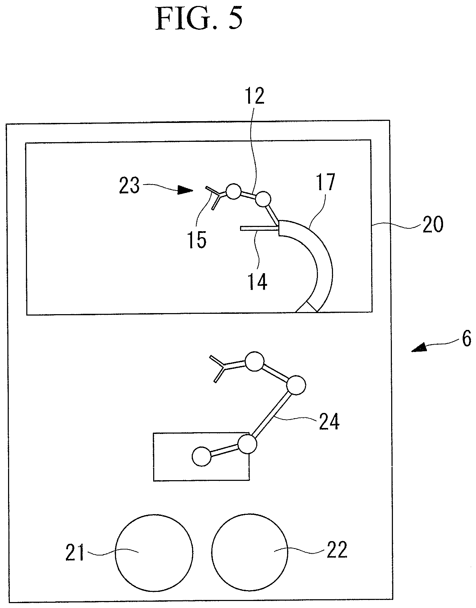

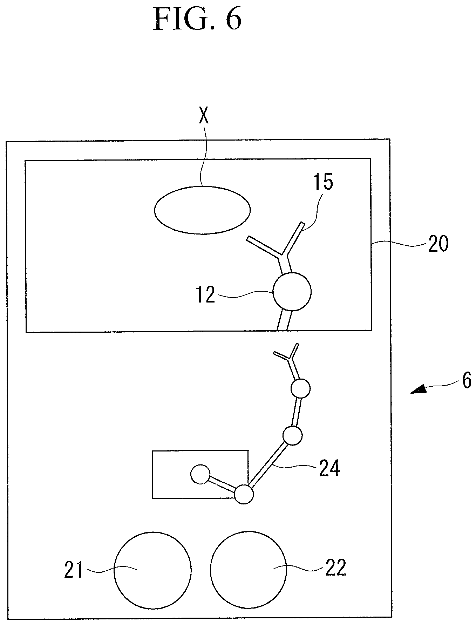

In this embodiment, although a case including the first robot arm 3, which supports the treatment unit 8 provided with the insertion section 11, the two manipulators 12 and 13, and the first imaging unit 14, the second treatment section 9, and the second robot arm 4, which supports the second treatment section 9, has been illustrated as an example, instead of this, as shown in Figs. 4 to 6, this embodiment may be applied to a case including only a first robot arm 3 that supports a treatment unit 23 provided with the insertion section 11, the single manipulator 12, and the first imaging unit 14.

In this case, a single operation input section 24 on the operation unit 6 may also be provided. In other words, in this case, when the field of view is switched to the global field of view of the endoscope 10, the robot arm 3 should be operated by means of the operation input section 24, and when the field of view is switched to the local field of view of the first imaging unit 14, the manipulator 12 should be moved by means of the operation input section 24.

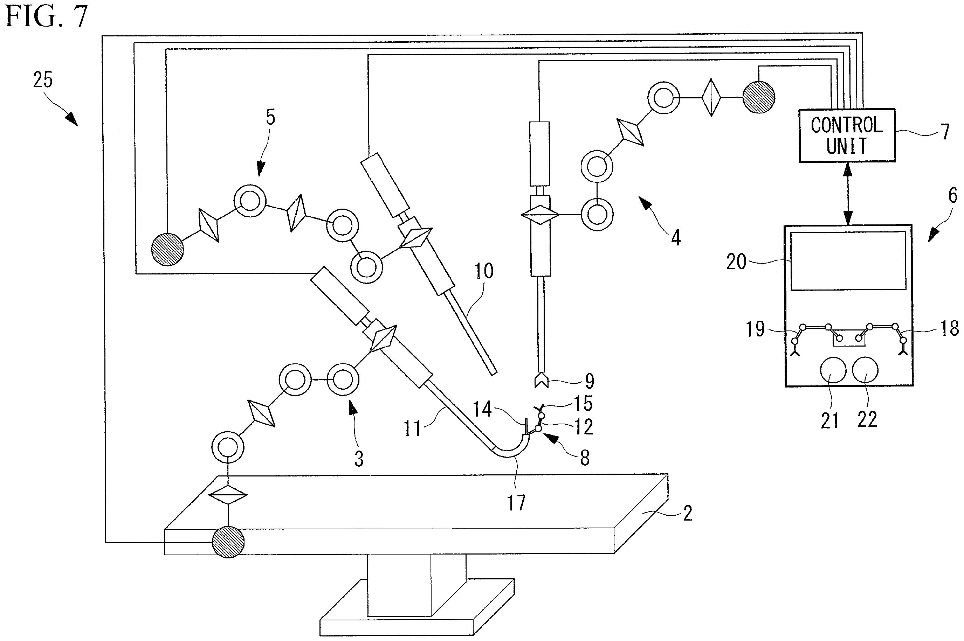

As shown in Fig. 7, a medical system 25 similar to that in Fig. 1, but not having the second manipulator 13 may be employed. In this case, treatment may be performed in the overview mode with the pair of robot arms, that is, the first robot arm 3 and the second robot arm 4, and in the local mode with only a single arm, that is, with only the single manipulator 12.

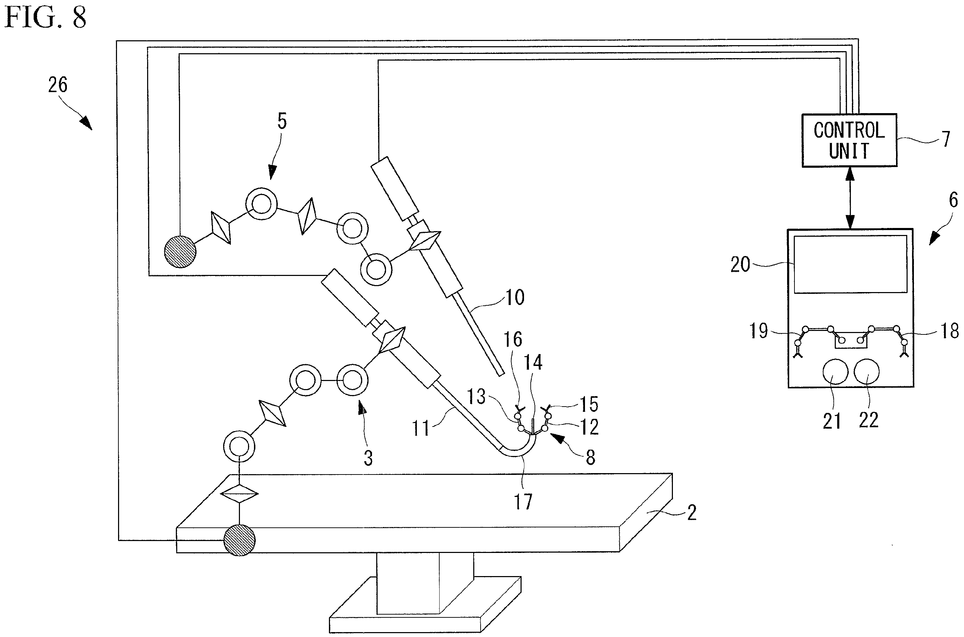

Conversely, as shown in Fig. 8, a medical system 26 similar to that in Fig. 1 but not having the second robot arm 4 may be employed. In this case, treatment may be performed in the overview mode with a single arm, that is, with only the first robot arm 3, and in the local mode with a pair of arms, that is, with the first manipulator 12 and the second manipulator 13.

In the local mode in which the video obtained by the first imaging unit 14 is displayed on the monitor 20, in some cases it is desired to minutely adjust the hand-eye-coordination; however, there is a problem in that when the mode is switched to the overview mode, such minute adjustment becomes difficult. In order to overcome this problem, the operation unit 6 is provided with an adjustment switch, and when this adjustment switch is depressed in the local mode, the screen is not switched while this adjustment switch is being depressed, and the control targets are switched to joints in the overview mode and are operated.

At this time, the first manipulator 12 and the second manipulator 13 are not moved, and only the bending section 17 and the joints of the robot arm 3 are moved.

Accordingly, hand-eye-coordination can be minutely adjusted without switching the image and the coordinate system.

### {Reference Signs List}

- 1, 25, 26: medical system
- 3: first robot arm
- 4: second robot arm
- 6: operation unit
- 7: control unit
- 9: second treatment section
- 10: endoscope (second imaging unit)
- 12: first manipulator
- 13: second manipulator
- 14: first imaging unit
- 15: first treatment section
- 16: third treatment section
- 18: first operation input section
- 19: second operation input section
- 20: monitor (display unit)
- 21: clutch
- 22: switching switch (switching unit)

## Claims

1. A medical system comprising:
a first robot arm provided, at a distal end thereof, with a first manipulator including a first treatment section at a distal end thereof and with a first imaging unit having a local field of view that includes a movable range of the first treatment section moved by the first manipulator;
a second imaging unit having a global field of view that is wider than the local field of view of the first imaging unit;
a display unit that displays video from the second imaging unit or the first imaging unit;
a switching unit that switches the video displayed on the display unit;
an operation unit for inputting an operation command for the first manipulator and the first robot arm; and
a control unit that controls the first manipulator and the first robot arm on the basis of the operation command input via the operation unit,
wherein the control unit controls the first robot arm and the first manipulator according to the operation command input via the operation unit when the switching unit switches to the video from the second imaging unit, and controls the first manipulator according to the operation command input via the operation unit when the switching unit switches to the video from the first imaging unit.

2. A medical system according to claim 1, further comprising:
a second robot arm provided with a second treatment section at a distal end thereof and having a movable range of the second treatment section within the global field of view,
wherein the control unit controls the first robot arm, the first manipulator, and the second robot arm according to the operation command input via the operation unit when the switching unit switches to the video from the second imaging unit.

3. A medical system according to claim 1 or 2, further comprising:
at a distal end of the first robot arm, a second manipulator provided with a third treatment section at a distal end thereof and having a movable range of the third treatment section within the local field of view,
wherein the control unit controls the first manipulator and the second manipulator according to the operation command input via the operation unit when the switching unit switches to the video from the first imaging unit.

4. A medical system according to claim 2 or 3, wherein
the operation unit is provided with a first operation input section that is operated by one hand of an operator and a second operation input section that is operated by the other hand of the operator; and
when the switching unit switches to the video from the second imaging unit, the control unit controls the first robot arm and the first manipulator according to the operation command input via the first operation input section, and controls the second robot arm according to the operation command input via the second operation input section.

5. A medical system according to claim 4, wherein when the switching unit switches to the video from the first imaging unit, the control unit controls the first manipulator according to the operation command input via the first operation input section, and controls the second manipulator according to the operation command input via the second operation input section.

6. A medical system according to one of claims 1 to 5, wherein, when the switching unit switches to the video from the second imaging unit, the control unit makes a coordinate system of the operation unit coincide with a fixed coordinate system in the second imaging unit.

7. A medical system according to one of claims 1 to 6, wherein, when the switching unit switches to the video from the first imaging unit, the control unit makes the coordinate system of the operation unit coincide with a fixed coordinate system in the first imaging unit.
